# EUROPÄISCHE PATENTANMELDUNG

(11) **EP 0 530 614 A1**
(43) Veröffentlichungstag der Anmeldung: **10.03.1993**
(21) Anmeldenummer: 92114285.7
(22) Anmeldetag: 21.08.1992
(51) Int. Cl.: C07H 21/00

(54) **Neuartige Antisense-Oligonukleotide mit durchgängiger Phosphoramidat-Internukleotidbindung**

(30) Priorität: 03.09.1991 DE 4129318
(71) Anmelder: BAYER AG, 51368 Leverkusen (DE)
(72) Erfinder: Löbberding, Antonius, Dr., W-5600 Wuppertal (DE); Mielke, Burkhard, Dr., W-5090 Leverkusen (DE); Springer, Wolfgang, Dr., W-5600 Wuppertal (DE); Stropp, Udo, Dr., W-5657 Haan (DE); Baumgarten, Jörg, Dr., W-5600 Wuppertal (DE); Rübsamen-Waigmann, Helga, Prof. Dr., W-6232 Bad Soden (DE); Biesert, Lothar, Dr., W-6000 Frankfurt/Main 50 (DE); Suhartono, Haryadi, Dr., W-6000 Frankfurt/Main 50 (DE)

(57) **Zusammenfassung**

Die Erfindung betrifft Oligonukleotide der allgemeinen Struktur I 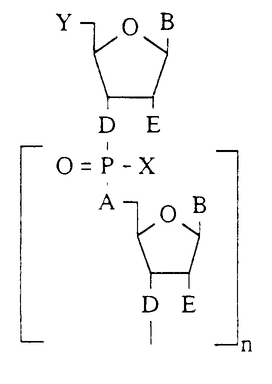
und ihre Verwendung bei der Behandlung genetisch bedingter Erkrankungen sowie bei der Abwehr körperfremder Nukleinsäuren.

## Beschreibung

Die gezielte Regulation unerwünschter Genexpression durch komplementäre Nukleinsäuren, sogenannte Antisense-Oligonukleotide, würde einen großen Fortschritt in der Chemotherapie darstellen. Sie könnte bei der Behandlung genetisch bedingter Erkrankungen sowie bei der Abwehr körperfremder Nukleinsäuren, z.B. nach einem Befall durch Bakterien, Pilze oder Viren von Bedeutung sein.

Das Antisense-Prinzip beruht auf der Fähigkeit der sogenannten Antisense-Oligonukleotide, mit der zu hemmenden mRNA zu hybridisieren und auf diese Weise die Proteinbiosynthese zu verhindern.

In der Literatur gibt es Belege für die Wirkung von Antisense-Oligonukleotiden. So wird u.a. ihre antivirale Wirkung z.B. gegen Retroviren wie HIV diskutiert (P.C. Hoyle, E.G. Cooper, Adv. Appl. Biotechnol. Ser. 2, 1989, 35).

Der Anwendung naturidentischer Phosphordiester-Oligonukleotide steht ihre hohe Sensitivität gegenüber enzymatischer Degradation durch körpereigene Endo- und Exonukleasen entgegen.

Gegenstand der vorliegenden Erfindung sind Antisense-Oligonukleotid-Analoga mit achiraler Phosphoramidat-Internukleotidbindung, die sich von 5'-Amino-2',5'-didesoxyfuranosen ableiten und am 5'-Ende durch Lipidreste modifiziert sein können. Diese Oligonukleotid-Analoga weisen eine erhöhte Resistenz gegen Endo- und Exonukleasen auf und zeigen antivirale Wirkungen.

Oligonukleotidanaloga, die eine oder mehrere Phosphorantidat-Internukleotidbindungen enthalten, sind vorbeschrieben (W. Bannwarth, Helv. Chim. Acta 71 (1988), 1517; M. Mag, J.W. Engels Nucleic Acids Res. 17 (1989), 5973; S.M. Gryaznov, N.I. Sokolova, Tetrahedron Lett. 31 (1990), 3205).

Diese Verbindungen weisen keine verbesserten Eigenschaften gegenüber nicht modifizierten Phosphordiester-Oligonukleotiden auf. Antivirale Aktivitäten dieser Verbindungen sind nicht bekannt.

Synthesen von Oligonukleotid-Sequenzen, die durchgängig Phosphoramidat-lnternukleotidbindungen enthalten, sind nicht beschrieben.

Die durchgängige Einführung von Phosphoramidat-Internukleotidbindungen führt jedoch zu ausreichend nukleasestabilen Molekülen mit antiviraler Wirkung. Auch der Einsatz dieser Strukturen als Gen-Sonden (Review: J.A. Matthews, L.J. Kricka, Analytical Biochemistry 169 (1988), 1) ist möglich.

Die Erfindung betrifft Oligonukleotide der allgemeinen Struktur I: 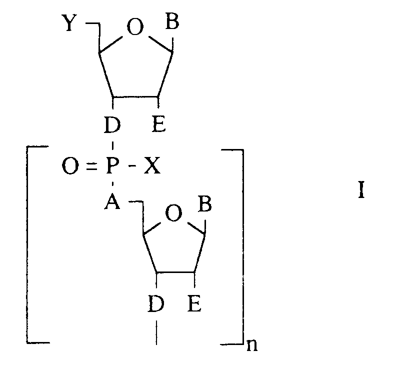
wobei
- A: durchgängig NH oder NR² sein kann,
- B: unabhängig voneinander eine natürlich vorkommende oder modifizierte Pyrimidin- oder Purinnukleobase oder auch OH, OR² oder H sein kann,
- D: O, S, NH oder NR² sein kann,
- E: unabhängig voneinander H, OH, OR², Azid oder Halogen
- X: O^{⊖ʼ} S^{⊖,} -OR², -SR² oder Alkyl.
- Y: -NH₂, -NHR²,-NR₂² oder 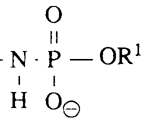
- R¹: steht für Alkyl, Aryl, Hetaryl, Aralkyl, Cycloalkyl, Lipid.
Unter Alkyl ist hier zu verstehen ein verzweigter oder unverzweigter Alkylrest mit 1 bis 30 C-Atomen. Bevorzugt sind Alkylreste mit 1 bis 18 C-Atomen und ganz besonders bevorzugt Alkylreste mit 6 bis 18 C-Atomen.
Unter Aryl ist hier z.B. Phenyl oder Naphthyl zu verstehen, gegebenenfalls substituiert, z.B. durch Halogen oder Nitro.
Unter Hetaryl ist z.B. Pyridyl, Acridinyl oder Carbazolyl zu verstehen.
Unter Aralkyl ist zu verstehen ein Aralkylrest mit 1 bis 18 C-Atomen im geradkettigen oder verzweigten Alkylteil und 6 bis 12 C-Atomen im Arylteil, wobei Aryl für Phenyl oder Naphthyl steht.
Unter Cycloalkyl ist ein cyclischer Alkylrest mit 3 bis 8 Kohlenstoffatomen zu verstehen, bevorzugt mit 3 bis 6 Kohlenstoffatomen, besonders bevorzugt Cyclopentyl und Cyclohexyl.
- R²: steht für Alkyl, Aryl, Aralkyl oder Cycloalkyl.
Unter Alkyl ist hier zu verstehen ein verzweigter oder unverzweigter Alkylrest mit 1 bis 20 C-Atomen. Bevorzugt sind Alkylreste mit 1 bis 10 C-Atomen, besonders bevorzugt Methyl, Ethyl, Propyl, Allyl, 2,2-Dimethylallyl, Benzyl.
Unter Aryl ist hier zu verstehen Phenyl, Naphthyl, gegebenenfalls substituiert, z.B. durch Halogen oder Nitro.
Unter Aralkyl ist hier zu verstehen ein Aralkylrest mit 1 bis 18 Kohlenstoffatomen im geradkettigen oder verzweigten Alkylteil und 6 bis 12 C-Atomen im Arylteil, wobei Aryl für Phenyl oder Naphthyl steht.
Unter Cycloalkyl ist hier ein cyclischer Alkylrest mit 3 bis 8 Kohlenstoffatomen zu verstehen, bevorzugt mit 3 bis 6 Kohlenstoffatomen, besonders bevorzugt Cyclopentyl und Cyclohexyl.
- n: 3 bis 50 sein kann.

Bevorzugt beträgt n 10 bis 30 und besonders bevorzugt 18 bis 28.

Die Synthese der aktivierten Monomeren erfolgt nach bekannten Verfahren (M. Mag. J.W. Engels, Nucleic Acids Res. 17 (1989), 5979).

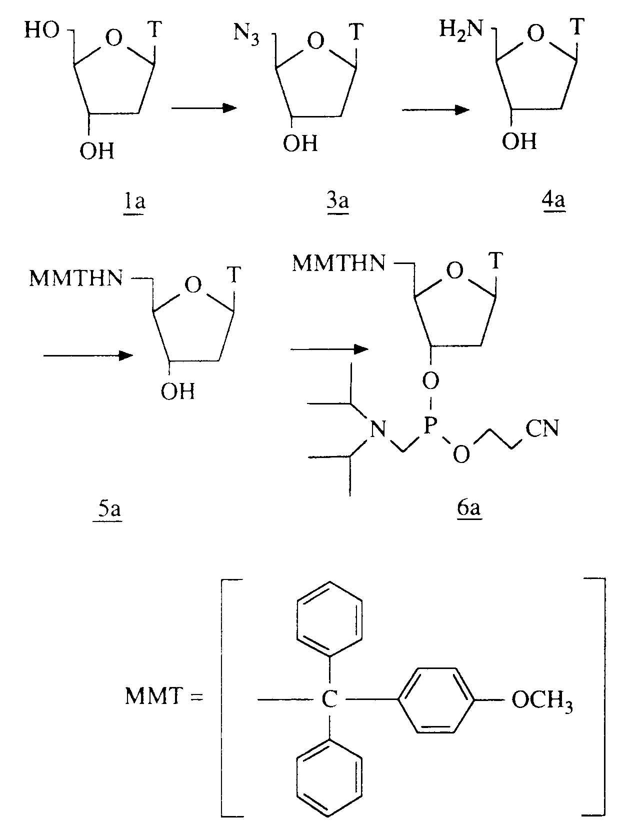

Thymidin wird mit Lithiumazid, Tetrabrommethan und Triphenylphosphin in Dimethylformamid zur Reaktion gebracht. Die Azidogruppe wird mit Triphenylphosphin in Pyridin zur Aminogruppe reduziert, die Aminogruppe nachfolgend mit der Monomethoxytritylgruppe geschützt. Das aktivierte Derivat 6a kann aus 5a mit Cyanethyl-N,N,N',N'-tetraisopropyl-phosphordiamidit erhalten werden.

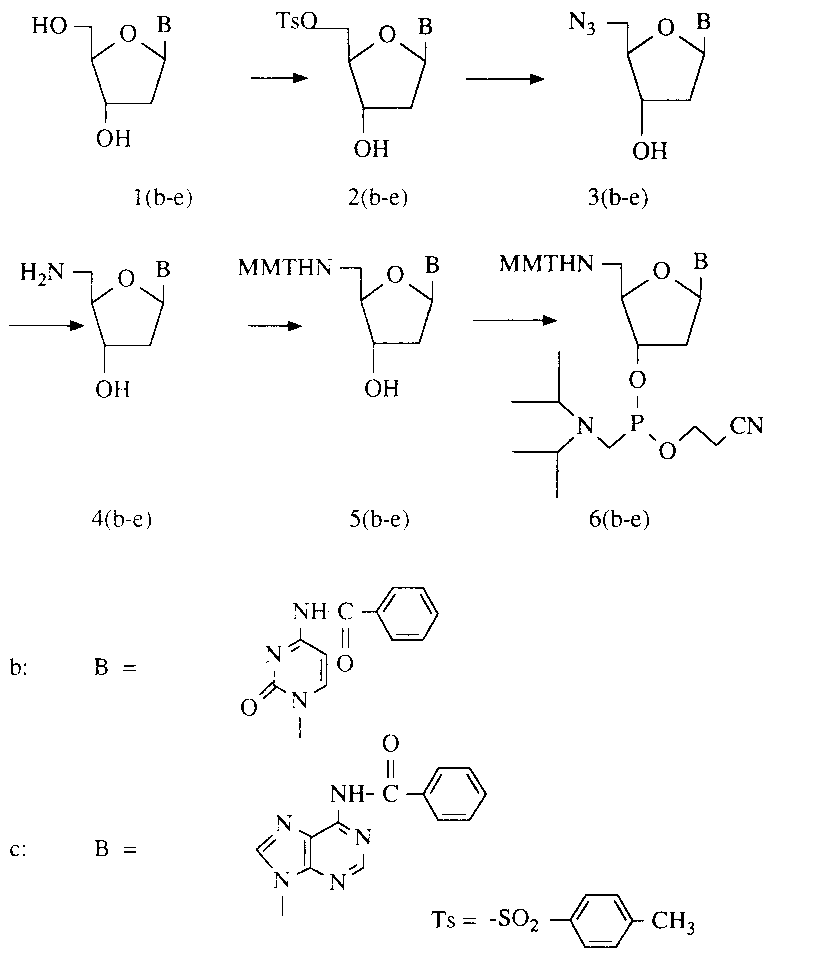

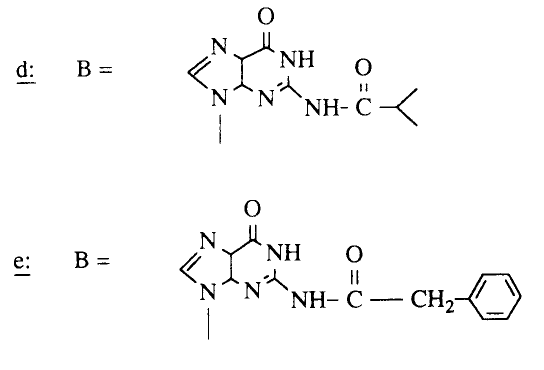

N⁴-Benzoyl-2'-desoxycytidin 1b. N⁴-Benzoyl-2'-desoxyadenosin 1c, N²-Isobutyryl-2'-desoxyguanosin 1d und N²-Phenylacetyl-2'-desoxyguanosin 1e werden nach der "Transient-Protection"-Methode hergestellt (G.S.Ti., B.L. Gaffney, R.A. Jones, J.Am. Chem. Soc. 104 (1982), 1316; F. Benseler, L.W. McLaughlin, Synthesis 1986, 45).

Aus den Bausteinen 1 (b-e) werden die Monotosylate 2 (b-e) gewonnen, die mit Lithiumazid in die 5'-Azidoverbindungen 3 (b-e) überführt werden.

Für die weiteren Reaktionsschritte wird der für das Thymidin-Derivat beschriebene Syntheseweg ausgenutzt.

Die Aktivierung der Lipidreste, z.B. Undecanol erfolgt analog durch Umsetzung mit Cyanethyl-N,N,N',N'-tetraisopropyl-phosphordiamidit, z.B. zu 7

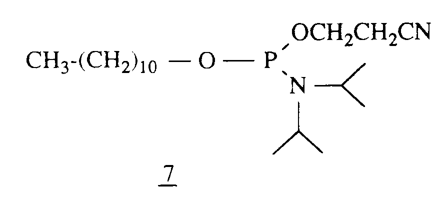

### Verknüpfung der 5,-Desoxyaminonukleotidbausteine zu Oligonukleotiden

Die Verknüpfung der Monomerbausteine zu Oligonukleotiden erfolgt vorzugsweise unter den Bedingungen der Festphasensynthese in Anlehnung an ein beschriebenes Verfahren (M. Mag, J.W. Engels, Nucl. Acids Res. 17, 5973 (1989); W. Bannwarth, Helv. Chim. Acta 71, 1517 (1988)).

Die erfindungsgemäßen Oligonukleotidanaloga werden im letzten Reaktionsschritt beispielsweise mit dem Phosphoramiditreagenz 7 verknüpft. Nach Abtrennung der Schutzgruppen sowie der Abspaltung der Sequenz von der festen Phase durch Basenbehandlung wird das Reaktionsprodukt vorzugsweise durch präparative HPLC isoliert, wobei das chromatographische Verhalten jeweils durch den Lipidrest bestimmt wird, der ein leichtes Abtrennen von Nebenprodukten erlaubt.
R_{NH}G_{NH}C_{NH}T_{NH}C_{NH}C_{NH}C_{NH}A_{NH}G_{NH}G_{NH}C_{NH}T_{NH}C_{NH}A_{NH}G_{NH}A_{NH}TC 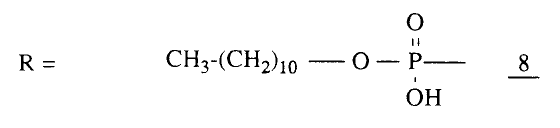
Die entsprechenden pharmazeutischen Zubereitungen enthalten neben den Phosphoramidat-Oligonukleotiden die für parenterale Zubereitungen üblichen Hilfsstoffe wie z.B. Puffer und/oder Stabilisatoren oder Liposomenformulierungen. Ferner ist eine topische Anwendung denkbar. Die hierfür einsetzbaren Zubereitungen sind z.B. Salben, Cremes, Lösungen oder Pflaster, die neben dem Wirkstoff die für diese Anwendung geeigneten pharmazeutischen Hilfsstoffe enthalten.

### Synthesebeispiele

### Beispiel 1:

### 5'-O-(4-Toluolsulfonyl)-N²-phenylacetyl-2'-desoxyguanosin

N²-Phenylacetyl-2'-desoxyguanosin (13,9 g; 36 mmol) und 4-Toluolsulfonsäurechlorid (21,0 g; 108 mmol) werden in wasserfreiem Pyridin (140 ml) 70 Min. bei Raumtemperatur gerührt. Danach wird bei 0°C Wasser (5 ml) zugegeben, 20 Min. bei Raumtemperatur nachgerührt, am Hochvakuum eingeengt, mehrfach mit Toluol co-destilliert, der Rückstand in Ethylacetat (150 ml) aufgenommen, je einmal mit 5 % NaHCO₃-Lösung und NaCl-Lösung (je 50 ml) gewaschen, die organische Phase getrocknet (MgSO₄) und eingeengt. Das Rohprodukt wird chromatographisch gereinigt (Dichlormethan/Methanol 15:1).
Ausbeute: 11`3g (58%).

### Beispiel 2:

### 5'-Azido-N²-phenylacetyl-2',5'-didesoxyguanosin

Eine Lösung von 5'-O-(4-Toluolsulfonyl)-N²-phenylacetyl-2'-desoxyguanosin (11,0 g; 20 mmol) in N,N-Dimethylformamid (120 ml) wird mit Lithiumazid (5,0 g; 100 mmol) versetzt und 7,5 h bei 50°C gerührt. Es wird abgekühlt, am Hochvakuum eingeengt, mehrfach mit Toluol co-destilliert, der Rückstand in Ethylacetat (500 ml) aufgenommen und je zweimal mit NaHCO₃-Lösung (je 60 ml) sowie NaCl-Lösung (je 100 ml) ausgeschüttelt. Die vereinigte wäßrige Phase wird mehrfach mit Ethylacetat rückextrahiert. Die vereinigten organischen Phasen werden getrocknet (MgSO₄) und eingeengt. Die Reinigung des Rohproduktes erfolgt chromatographisch (Dichlormethan/Methanol 13:1 → 10:1).
Ausbeute: 8,0 g (95 %).

### Beispiel 3:

### 5'-Amino-N²-phenylacetyl-2',5'-didesoxyguanosin

5'-Azido-N²-phenylacetyl-2',5'-didesoxyguanosin (8,2 g; 29 mmol) und Triphenylphosphin (7,9 g; 30 mmol) werden in Pyridin (150 ml) gelöst und 16 h bei Raumtemperatur belassen. Dann wird Wasser (25 ml) zugegeben und 2 h nachgerührt. Die Reaktionslösung wird auf Wasser (200 ml) gegossen, abfiltriert und die wäßrige Phase zweimal mit Ethylacetat (je 100 ml) extrahiert. Die vereinigte organische Phase wird noch fünfmal mit Wasser (je 100 ml) extrahiert. Die vereinigte wäßrige Phase wird gefriergetrocknet und ergibt das Rohprodukt als gelbliches Pulver, das sofort weiter umgesetzt wird.
Ausbeute: 7.1 g (92 %)

### Beispiel 4:

### 5'-(4-Methoxytriphenylmethyl)amino-N²-phenylacetyl-2',5'-didesoxyguanosin

5'-Amino-N²-phenylacetyl-2',5'-didesoxyguanosin 7,1 g; 18,5 mmol) wird in Pyridin (200 ml) gelöst. mit Chlor-(4-methoxy)-triphenylmethan (17,0 g; 55,5 mmol). 4-(N,N-Dimethylamino)-pyridin (490 mg; 3,8 mmol) und Triethylamin (2,7 ml; 19 mmol) versetzt und 2,5 h bei Raumtemperatur gerührt. Sodann wird am Hochvakuum eingeengt, mehrfach mit Toluol co-destilliert, der Rückstand in Dichlormethan (500 ml) aufgenommen, je zweimal mit NaHCO₃-Lösung und NaCl-Lösung ausgeschüttelt, die organische Phase getrocknet (MgSO₄) und eingeengt. Eine chromatographische Reinigung schließt sich an (Dichlormethan/Methanol 20:1 → 10:1).
Ausbeute: 2.4 g (20 %).

### Beispiel 5:

### 5'-(4-Methoxytriphenylmethyl)amino-N²-phenylacetyl-2',5'-didesoxyguanosin-3'-0-(2-cyanethyl-N,N-diisopropylamino)phosphit

5'-(4-Methoxytriphenylmethyl)amino-N²-phenylacetyl-2',5'-didesoxyguanosin (2,2 g; 3,3 mmol) und Tetrazol (176 mg; 2,5 mmol) werden unter strengstem Luft- und Feuchtigkeitsausschluß in absolutem Dichlormethan/Acetonitril (1:1; 70 ml) gelöst, mit 2-Cyanethyl-N,N,N',N'-tetraisopropyl-phosphordiamidit (1,65 ml; 5,3 mmol) versetzt und 1,5 h bei Raumtemperatur gerührt. Nach vollständiger Umsetzung wird Butanol (3 ml) zugesetzt, 10 Min. nachgerührt, mit Dichlormethan (500 ml) verdünnt, zügig je einmal mit 5 % NaHCO₃-Lösung (150 ml) und NaCl-Lösung (250 ml) extrahiert, getrocknet (MgSO₄) und eingeengt. Das Rohprodukt wird in Dichlormethan/Ether (1:1, 14 ml) gelöst und in trockeneis-gekühltes n-Pentan (450 ml) präzipitiert.
Ausbeute: 1.67 g (59 %).

### Beispiel 6:

### Undecan-1-0-(2-cyanethyl-N,N-diisopropylamino)phosphit

Zur Lösung von Undecanol (1.72 g. 10 mmol), Diisopropylamin (0,5 g, 5 mmol) und Tetrazol (350 mg. 5 mmol) in 30 ml Dichlormethan wird unter Argon innerhalb von 20 Min. Bis-(diisopropylamino)-(2-cyanoethoxy)-phosphin (3,3 g, 11 mmol) unter Rühren zugefügt.

Nach Beendigung der Reaktion wird mit 10 %iger kalter Natriumcarbonatlösung ausgeschüttelt. Die organische Phase wird eingedampft und das Produkt an Kieselgel chromatographiert.
Ausbeute: 3,3 g, (73 %).

### Beispiel 7:

Synthese der nachfolgend aufgeführten Beispielsequenz:
R_{NH}G_{NH}C_{NH}T_{NH}C_{NH}C_{NH}C_{NH}A_{NH}G_{NH}G_{NH}C_{NH}T_{NH}C_{NH}A_{NH}G_{NH}A_{NH}TC 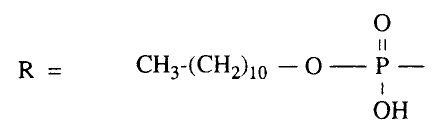
Die Synthese wurde an einem Applied Biosystems, 380 B DNA-Syntheseautomaten durchgeführt. Als feste Phase diente ein kommerziell erhältlicher (Applied Biosystems) 10 µmol (oder 1 µmol) control-poreglass-Träger an dem über die 3'-Hydroxyfunktion 4,4'-Dimethoxytrityl-geschütztes 2-Desoxycytidin angebunden ist. Die 4,4'-Dimethoxytritylschutzgruppe wird durch Behandlung mit 2,5 %iger Dichloressigsäure in Methylenchlorid abgespalten und die überschüssige Säure und die Schutzgruppe im Anschluß mit Acetonitril ausgewaschen. Die Kettenverlängerung erfolgt durch Zufügen einer 0,1 M Lösung von 5'-NH-Monomethoxytrityl-geschütztem Nukleotidbaustein 6a in Acetonitril in Gegenwart von Tetrazol. Im Anschluß erfolgt die Oxidation zum pentavalenten Phosphor durch Behandlung mit einer 0,2 M Jod-Lösung in Pyridin/H₂O/THF/1:1:8. Zur Vermeidung von Fehlsequenzen wird nachfolgend nicht umgesetzte 5'-Hydroxy- oder 5'-Aminofunktion (bei den nachfolgenden Zyklen) durch Behandlung mit Acetanhydrid/Dimethylaminopyridin in Pyridin/Acetonitril blockiert.

Der nächste Reaktionszyklus beginnt mit der Abspaltung der Monomethoxytritylschutzgruppe durch Behandlung mit 2,5 %iger Dichloressigsäure. Die Umsetzung mit den nachfolgenden 5'-Aminonukleotidbausteinen 6 erfolgt analog wie bereits oben beschrieben.

Im letzten Reaktionsschritt wird eine 0,1 M Lösung des Undecanol-β-cyanethyl-phosphoramidit-Reagenzes 7 in Acetonitril in Gegenwart von Tetrazol nach saurer Abspaltung der letzten Monomethoxytritylschutzgruppe angekoppelt. Nach Jod-Oxidation erfolgt die Abspaltung vom polymeren Träger und die Abtrennung der Schutzgruppen durch Behandlung mit konzentriertem Ammoniak bei 55°C über 16 h.

Das Reaktionsprodukt wird durch präparative HPLC an einer RP 18-Säule mit einem linear ansteigendem Gradienten von Acetonitril in 0,1 M Triethylammoniumacetat isoliert; Ausbeute: 2,8 mg (4,8 %).

### Nukleasestabilität von Oligonukleotid-Amidaten

Neben der Kettenlänge. Sequenz und Zellpermeabilität spielt die Nukleaseresistenz eine wichtige Rolle für die biologische Wirkung von Antisense-Oligonukleotiden. Die synthetisierten Oligonukleotide mit 5'-Amidat-Intenukleotidbindung wurden deshalb in Bezug auf ihre Nukleasestabilität mit natürlichen Oligonukleotid-Diestetn verglichen.

Die Antisense-Oligonukleotid-Diester und Amidate wurden hierzu am 5' Ende mit ³²P-ATP radioaktiv markiert und ihre Degradation mit verschiedenen definierten Nukleasen und Zellextrakten durch Polyacrylamidgelelektrophorese und Autoradiographie überprüft und durch die Isolierung der Degradationsprodukte aus dem Gel im Scintillationscounter quantitativ bestimmt.

Natürliche Oligonukleotid-Diester weisen eine nur geringe Nukleasestabilität auf. Sie werden innerhalb von 30 Minuten bis 1 Stunde vollständig degradiert. Oligonukleotid-Amidate sind dagegen 10 bis 100-fach resistenter gegenüber Nukleasen und eignen sich deshalb besonders gut für den Einsatz als antivirale Antisense-Oligonukleotid-Inhibitoren.

### Antivirale Wirkungen:

### In-Vitro-Translationstest

Die antivirale, translationsinhibierende Aktivität der Amidate wurde in einem zellfreien in vitro Translationstest gezeigt. Hierzu wurde in vitro synthetisierte TAR-tat-mRNA in ein Kaninchen-Retikulozyten-Translationssystem gegeben und die tat-Proteinsynthese mit und ohne Oligonukleotid quantitativ erfaßt.

Im Detail: 1 µg in vitro synthetisierte TAR-tat-mRNA wird mit einer gewünschten Menge Oligonukleotid (0.2-2 µg) versetzt in einem Volumen von 2,5 µl. Zur Translation wird 10 µl vorinkubierter Translationsmix (Fa. Promega) mit radiaktivem ³⁵S-Cystein auf die 2,5 µl pipettiert und 90 min. bei 30°C inkubiert. Ein Aliquot des Ansatzes wird entnommen und mit SDS-Auftragspuffer erhitzt und über ein 6-20 % diskontinuierliches SDS-PAGE aufgetrennt. Nach Fixierung und Trocknung wird das Gel autoradiographiert und die erhaltenen tat-Protein-Banden densitometrisch ausgewertet.

Resultat: die getesteten Amidate zeigen eine 3 mal stärkere Inhibition der Translation des tat-Proteins als sequenzgleiche Diester-Oligonukleotide in verschiedenen Konzentrationen.

### Antivirale Wirkung im Zellkulturtest

Die Prüfung der Phosphoramidat-Oligonukleotide wurde an den nachfolgend aufgeführten Virus-Isolaten durchgeführt:
- HIV-1_{D34}: Isolierung durch Dr. von Briesen und Prof. Dr. H. Rübsamen-Waigmann, Georg-Speyer-Haus, Frankfurt am Main, 1985.
- HIV-2_{ROD}: Isolierung durch Pasteur-Institut, Paris, 1986.

Als Testsystem dienten periphere Blutlymphozyten (PBL) aus HIV-seronegativen Spendern, die mit Phytohämagglutinin voraktiviert wurden. Die Substanzen wurden unmittelbar nach der Infektion zur Zellkultur gegeben. Die Zellsuspensionen wurden mit unterschiedlichen Konzentrationen der Verbindungen 4-6 Tage im Brutschrank kultiviert. Der Nachweis der Virusreplikation erfolgte über die lichtmikroskopische Auswertung der HIV-induzierten Synzytienbildung.

**Tabelle 1:**

| Hemmung der Virus-indizierten Synzytienbildung in Gegenwart von Phosphoramidat-Oligonukleotid 8 | | | | |
|---|---|---|---|---|
| Versuch | HIV-1 | | HIV-2 | |
| | µg/ml | µmol/l | µg/ml | µmol/l |
| 1 | 5-10 | 0,9-1,9 | - | - |
| 2 | 2-5 | 0,4-0,9 | 15-25 | 2,8-4,7 |
| 3 | 2-5 | 0,4-0,9 | 15-25 | 2,8-4,7 |
| 4 | 5-10 | 0,9-1,9 | - | - |

Die Testsubstanz hemmt die HIV-1-induzierte Synzytienbildung deutlich in Konzentration oberhalb von 2-5 µg/ml, d.h. 0,4-0,9 µmol.
Die HIV-2-induzierte Synzytienbildung wird dagegen nur geringfügig bzw. deutlich weniger stark inhibiert.

## Patentansprüche

1. Oligonukleotide der allgemeinen Struktur I: 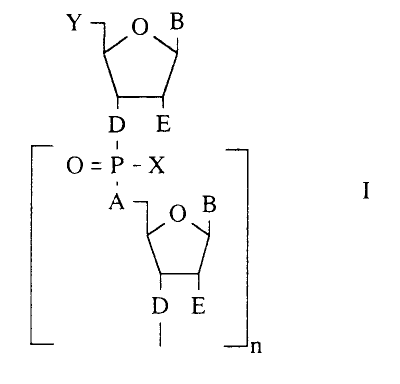 wobei
A durchgängig NH oder NR² sein kann,
B unabhängig voneinander eine natürlich vorkommende oder modifizierte Pyrimidin- oder Purinnukleobase oder auch OH, OR² oder H sein kann,
D O, S, NH oder NR² sein kann,
E unabhängig voneinander H, OH, OR², Azid oder Halogen sein kann,
X -O^{⊖,} S^{⊖,} -OR², -SR² oder Alkyl,
Y -NH₂, -NHR², -NR₂² oder 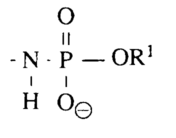
R¹ Alkyl, Aryl, Aralkyl, Cycloalkyl, Lipid,
R² Alkyl, Aryl, Aralkyl oder Cycloalkyl sein kann,
n 3 bis 50 sein kann.

2. Oligonukleotide gemäß Anspruch 1, wobei n 10 bis 30 ist.

3. Oligonukleotide gemäß Anspruch 1, wobei n 18 bis 28 ist.

4. Arzneimittel enthaltend ein oder mehrere der Oligonukleotide gemäß den Ansprüchen 1 bis 3.
